# EUROPEAN PATENT SPECIFICATION

(11) **EP 2 153 856 B1**
(45) Date of publication and mention of the grant of the patent: **13.01.2016**
(21) Application number: 08014343.1
(22) Date of filing: 12.08.2008
(51) Int. Cl.: A61M 5/50, A61M 5/34

(54) **Auto disable device for syringes**
Selbstsperrvorrichtung für Spritzen
Dispositif à désactivation automatique pour seringues

(43) Date of publication of application: 17.02.2010
(73) Proprietor: Abu Dhabi National Industrial Projects Co., Abu Dhabi (AE)
(72) Inventor: Kakish, Amer F.A., Abu Dhabi (AE); Al Omari, Osama Y.T., Abu Dhabi (AE)
(74) Representative: Paglia, Pietro

(56) References cited:
- WO-A-92/04064
- WO-A-2006/066336
- US-A- 2 902 995
- US-A- 3 491 757
- US-A- 5 613 951

## Description

### Field of the Invention

The invention pertains to devices for preventing the reuse of sterile single use syringes.

### Background of the Invention

It is known to provide a hypodermic syringe with a device that prevents it from being used more than a single time. Such devices are typically installed in the syringe barrel between the piston and the outlet end of the barrel and prevent the syringe from being refilled. An example is shown in Meyer et al., US 5,613,951. Since the devices are internal to the syringe barrel, either the barrel is sized and configured to fit a device of a specific diameter, or the devices are made in different sizes to fit the barrels of syringes of different sizes. Further, because of the requirement that the devices fit inside the barrel, there is a practical lower limit to the size, of syringe in which such devices can be installed. WO 92/04064 (Butler) discloses a single use syringe having a salve assembly for attachment to syringe barrel with a resiliently-deformable valve member. The valve member is moveable within a sleeve to engage a sealing surface and close the valve opening, preventing reuse.

### Summary of the Invention

The invention provides an anti-reuse device for a syringe, the device being external to the syringe. The device is an auto-disable device that is automatically activated and remains effective from the time that the injection of liquid from the syringe has commenced.

The auto-disable device comprises an assembly made of a barrel adapter and a needle hub. The barrel adapter is configured to attach to the outlet end of the barrel of a syringe, the attachment being external to the barrel. The needle hub is affixed to the barrel adapter and has a fixed hypodermic needle, which can be of various sizes as required. The assembly has a cavity with a passage for the flow of liquid from the assembly inlet to the needle. The device includes an elastically-deformable sealing ring in the cavity having an opening therein. A sealing member in the cavity has a head and a shaft, the sealing member being moveable in the cavity in a longitudinal direction from a first position in which the head of the sealing member is on a side of the ring facing the inlet of the assembly to a second position in which the head of the sealing member is on a side of the sealing ring facing the needle. The sealing ring is configured to stop movement of the sealing member from the second position to the first position and to form a seal with the head, when the sealing member is in the second position, against a flow of liquid in the direction of the inlet end of the assembly. Being external to the barrel of the syringe, the auto-disable device can be used with syringes of different sizes. There are therefore no practical limitations to the syringe barrel dimensions. The device can be conveniently attached to syringes that are intended for single use, to convert them into auto-disable syringes, provided that the outer luer cone of the syringe barrel is adjusted to permit locking of the device to the barrel. The device disables the syringe by means of the expulsion of liquid from the syringe in the course of doing an injection, without a further action to disable the syringe. Thus the term, auto-disable.

The auto-disable device, which includes the needle, can be assembled with the syringe at the factory and delivered as a unit, or, alternatively, the device can be provided separate from the syringe and be attached to it by a medical practitioner prior to use.

These and other features of the invention will be apparent from the following description and drawings of the preferred embodiments.

### Brief Description of the Drawings

Figure 1 is a longitudinal cross-sectional view of the auto-disable device of the invention attached to a sterile single use hypodermic syringe.
Figure 2 is a partial longitudinal cross-sectional view of the auto-disable device, with the moveable sealing member in the sealing position.
Figure 3 is a cross-sectional view on the line 3-3 of Figure 1.
Figure 4 is a cross-sectional view on the line 4-4 of Figure 1.

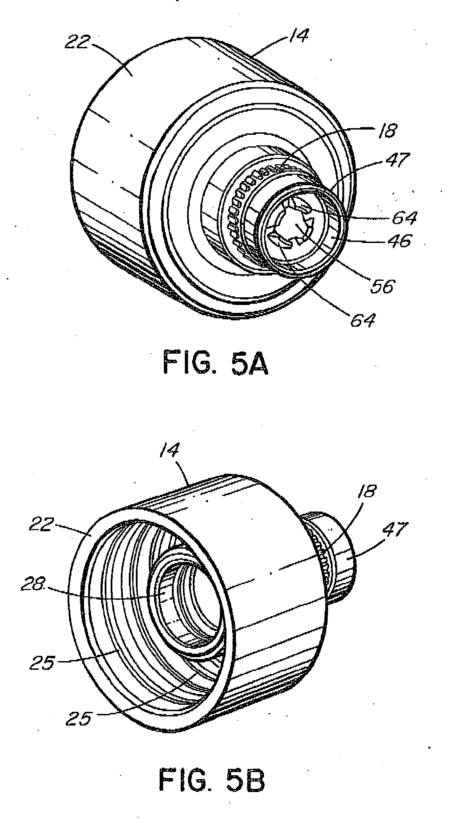

Figures 5A and 5B are perspective views of the barrel adapter portion of the auto-disable device assembly.

### Description of the Preferred Embodiments

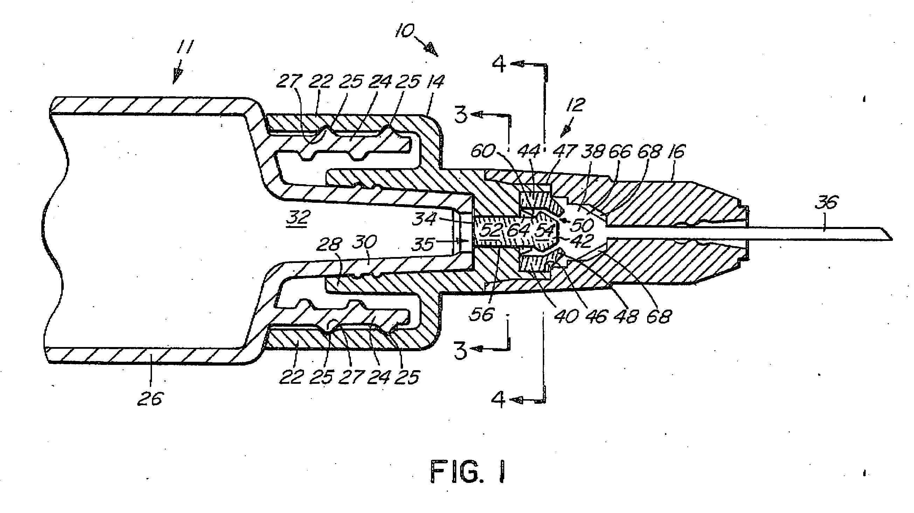

The auto-disable device 10 is shown in Figure 1 attached to a sterile single use hypodermic syringe 11. The device 10 has an assembly 12 formed of a barrel adapter 14 and a needle hub 16. The barrel adapter and hub attach securely together by means of circumferentially-disposed ribs 18 on the barrel adapter which interfit tightly with mating grooves 20 on the needle hub. A hypodermic needle 36 is affixed to the distal end of the needle hub.

The barrel adapter 14 has an outer circumferential wall 22 for locking engagement with a luer lock cone 24 of the barrel 26 of the syringe. A number of circumferential grooves 25 on the inner surface of the wall 22 interfit with circumferential ridges 27 on the outer surface of the luer lock cone 24 of the barrel, providing a snap-on fit. The barrel adapter 14 has an inner circumferential wall 28 for mating engagement with the outlet cone 30 at the outlet end 32 of the barrel. The barrel adapter 14 has an inlet 34 at an inlet end 35 thereof. The inlet 34 aligns with the barrel outlet at the distal end of the barrel outlet cone 30.

The assembly 12 has a cavity 38 extending longitudinally therethrough from the inlet 34 to the needle 36.

A sealing ring 40 and a moveable sealing member 42 are positioned in the cavity 38. The sealing ring has an annular base portion 44 which fits snugly against the inner surface 46 of the ring-retaining portion 47 of the barrel adapter, and an elastically-deformable, inwardly-extending sealing portion 48 having an opening 50 therein. The moveable sealing member 42 has a shaft portion 52 and a head portion 54.

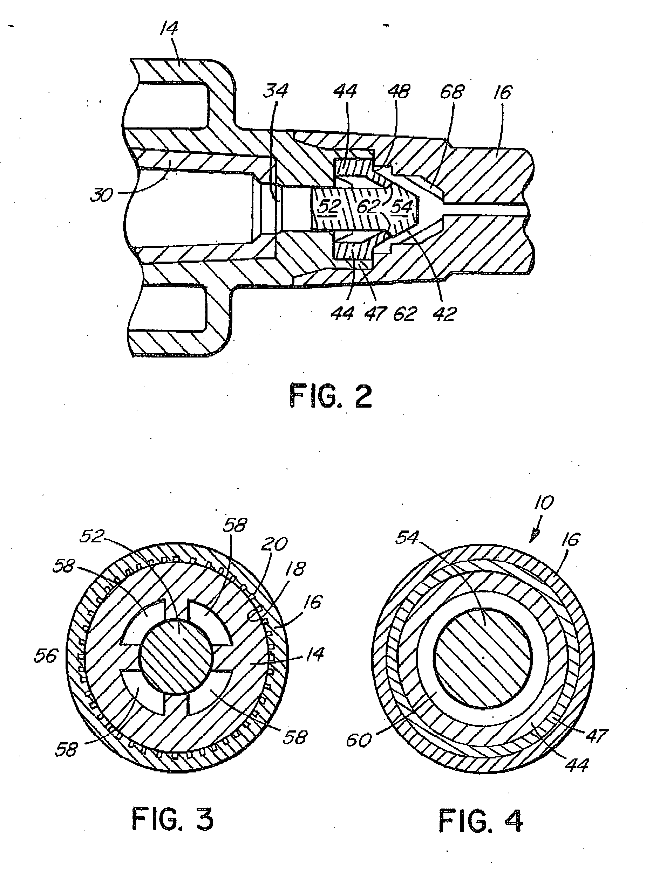

The barrel adapter 14 has a cylindrical guiding portion 56 which receives the shaft portion 52 of the sealing member 42. Four channels 58, best seen in Figure 3, extend along the sides of the guiding portion 56 between the inlet 34 of the barrel adapter and a section 60 of the cavity adjacent to the sealing ring. These channels permit the flow of liquid from the inlet 34 to the section 60 of the cavity 38 and also enable syringe filling.

The head 54 of the moveable sealing member 42 has a diameter larger than the diameter of the opening 50 of the sealing ring 40. The sealing portion 48 of the sealing ring is angled in the direction of the needle 36 and is elastically deformable such that the head 54 of the moveable sealing member can pass through the opening 50 in the direction of the needle under the pressure of liquid caused when the piston in the syringe is moved in the direction of the needle.

The barrel adapter 14 has four guiding lugs 64 which extend into the cavity section 60 adjacent to the guiding portion 56 of fhe barrel adapter. These lugs support the moveable sealing member 42 in its movement in the direction of the needle.

The cavity 38 includes a section 66 within the needle hub into which the head 54 of the moveable sealing member 42 moves. Channels 68 are provided in the wall of the cavity section 66, adjacent to the inward end of the needle, permitting the flow of liquid past the head 54 of the sealing member 42 when the head is in the cavity section 66.

For the initial filling of the syringe, the sealing member 42 is in a position on the side of the sealing member facing the inlet 34 of the auto-disable device 10. Liquid can then be drawn into the barrel of the syringe by movement of the syringe piston in the direction away from the needle, the liquid passing through the needle into the cavity 38, through the opening 50 in the sealing ring 40, through the channels 58 in the barrel adapter 14 and out through the inlet 34 into the syringe barrel 26. When the syringe piston is then pressed in the direction of the needle to deliver the liquid, the liquid flows from the barrel into the inlet 34 and through the channels 58, pressing against the inlet side of the sealing portion 48 of the ring 40 and against the inlet end of the moveable sealing member 42. This causes the opening 50 of the sealing ring 40 to enlarge and permit the head 54 of the moveable sealing member to be pushed through the opening and also permit the liquid to flow through the opening 50 in the direction of the needle, through the channels 68 and out through the needle.

Movement of the moveable sealing member 42 in the opposite direction is stopped by the engagement of the sealing portion 48 of the ring 40 with the head 54 of the moveable sealing member. As shown in Figure 2, the sealing portion 48, and more specifically its sealing surface 62 around the opening 50, seals against the head 54, stopping any flow of fluid in the direction of the inlet 34 of the assembly.

The components of the auto-disable device may be made of any suitable materials. Such suitable materials include polystyrene, polypropylene or polyethylene for the barrel adapter 14 and for the needle hub 16, acrylonitrile butadiene styrene (ABS) for the moveable sealing member 42, thermoplastic elastomer (TPE) for the seating ring 40 and stainless steel for the needle.

Although the invention has been described in terms of specific embodiments, it is not intended that the invention be limited to those embodiments. Various modifications within the scope of the invention will be apparent to those skilled in the art. For example the barrel adapter can be a luer slip barrel adapter, or be shaped to attach to various kinds of fittings on syringe barrels. The scope of the invention is defined by the claims that follow.

## Claims

1. An auto-disable device (10) for use with a sterile single use hypodermic syringe (11) comprising:
(a) a barrel adapter (14) configured to attach to an outlet end (32) of a barrel (26) of the syringe, said attachment being external to the barrel;
(b) a needle hub (16) affixed to the barrel adapter (14) and having a hypodermic needle (36) affixed thereto;
(c) the barrel adapter (14) and the needle hub (16) forming an assembly (12) having a longitudinal axis, the assembly having a cavity (38) with a passage for the flow of a liquid from an inlet (34) of the assembly to the needle (36), the cavity (38) having a section (66) thereof that is within the needle hub (16) and outside the barrel adaptor (14), the barrel adaptor having a cylindrical ring-retaining portion (47) at an end thereof that engages the needle hub (16);
(d) an elastically-deformable sealing ring (40) in the cavity (38), the sealing ring (40) having an annular base portion (44) and an inwardly-extending sealing portion (48) having an opening (50) therein, the annular base portion (44) being fitted into the ring-retaining portion (47) of the barrel adaptor (14);
(e) a sealing member (42) in the cavity (38) having a head (54) and a shaft (52), the sealing member (42) being moveable in the cavity (38) in a longitudinal direction from a first position in which the head (54) of the sealing member (42) is on a side of the sealing portion (48) of the ring facing the inlet (34) of the assembly (12) to a second position in which the head (54) of the sealing member (42) is on a side of the sealing portion (48) of the ring facing the needle (36) and within the section (66) of the cavity (38) that is within the needle hub (36); and
(f) the sealing portion (48) of the ring (40) being configured to stop movement of the sealing member (42) from the second position to the first position and to form a seal with the head (54), when the sealing member (42) is in the second position, against a flow of liquid in the direction from the needle to the inlet (34) of the assembly.

2. An auto-disable device (10) according to claim 1, wherein the barrel adapter (14) is a luer lock barrel adapter.

3. An auto-disable device (10) according to claim 1, wherein the barrel adapter (14) is a luer slip barrel adapter.

4. An auto-disable device (10) according to any preceding claim, further comprising interlocking ribs (18) and grooves (20) to attach the barrel adapter (14) and needle hub (16) together.

5. An auto-disable device (10) according to claim 1, wherein the barrel adapter (14) includes an outer circumferential wall (22) for locking engagement with a luer cone (24) of the syringe barrel (26).

6. An auto-disable device (10) accordingly to claim 1, wherein the barrel adapter (14) includes an inner circumferential wall (28) for mating engagement with an outlet cone (30) of the syringe barrel (26).

7. An auto-disable device (10) according to any preceding claim, in combination with a syringe (11).

## Patentansprüche

1. Selbstsperrvorrichtung (10) für den Einsatz mit einer sterilen Einweginjektionsspritze (11), umfassend:
(a) einen Körperadapter (14), welcher konfiguriert ist, um an einem Auslassende (32) eines Körpers (26) der Spritze angebracht zu werden, wobei die Anbringung außen an dem Körper erfolgt;
(b) einen Nadelansatz (16), welcher am Körperadapter (14) befestigt ist und eine daran befestigte hypodermische Nadel (36) aufweist;
(c) wobei der Körperadapter (14) und der Nadelansatz (16) einen Zusammenbau (12) mit einer Längsachse ausbilden, wobei der Zusammenbau eine Ausnehmung (38) mit einem Durchgang für den Fluss einer Flüssigkeit von einem Einlass (34) des Zusammenbaus zu der Nadel (36) aufweist, wobei die Ausnehmung (38) einen Abschnitt (66) davon aufweist, welcher in dem Nadelansatz (16) und außerhalb des Körperadapters (14) liegt, wobei der Körperadapter einen zylindrischen ringzurückhaltenden Abschnitt (47) an einem seiner Enden aufweist, welches mit dem Nadelansatz (16) in Eingriff steht;
(d) einen elastisch verformbaren Dichtungsring (40) in der Ausnehmung (38), wobei der Dichtungsring (40) einen ringförmigen Grundabschnitt (44) und einen sich nach innen erstreckenden Dichtungsabschnitt (48) mit einer Öffnung (50) darin aufweist, wobei der ringförmige Grundabschnitt (44) in den ringzurückhaltenden Abschnitt (47) des Körperadapters (14) eingepasst ist;
(e) ein Dichtungselement (42) in der Ausnehmung (38), das einen Kopf (54) und einen Schaft (52) aufweist, wobei das Dichtungselement (42) in der Ausnehmung (38) in einer Längsrichtung von einer ersten Position, in welcher sich der Kopf (54) des Dichtungselements (42) auf einer Seite des Dichtungsabschnitt (48) des Rings befindet, welche dem Einlass (34) des Zusammenbaus (12) zugewandt ist, zu einer zweiten Position bewegbar ist, in welcher sich der Kopf (54) des Dichtungselements (42) auf einer Seite des Dichtungsabschnitts (48) des Rings, welche der Nadel (36) zugewandt ist, und innerhalb des Abschnitts (66) der Ausnehmung (38) befindet, welcher innerhalb des Nadelansatzes (36) liegt; und
(f) wobei der Dichtungsabschnitt (48) des Rings (40) konfiguriert ist, um Bewegung des Dichtungselements (42) von der zweiten Position in die erste Position zu stoppen und um mit dem Kopf (54) eine Dichtung gegen einen Fluss von Flüssigkeit in die Richtung von der Nadel zu dem Einlass (34) des Zusammenbaus auszubilden, wenn das Dichtungselement (42) sich in der zweiten Position befindet.

2. Selbstsperrvorrichtung (10) nach Anspruch 1, wobei der Körperadapter (14) ein Luer-Lock-Körperadapter ist.

3. Selbstsperrvorrichtung (10) nach Anspruch 1, wobei der Körperadapter (14) ein Luer-Slip-Körperadapter ist.

4. Selbstsperrvorrichtung (10) nach einem beliebigen der vorangehenden Ansprüche, des Weiteren umfassend ineinandergreifende Rippen (18) und Nuten (20), um den Körperadapter (14) und den Nadelansatz (16) gemeinsam anzubringen.

5. Selbstsperrvorrichtung (10) nach Anspruch 1, wobei der Körperadapter (14) eine äußere Umfangswand (22) zum verriegelnden Eingriff mit einem Luer-Kegel (24) des Spritzenkörpers (26) umfasst.

6. Selbstsperrvorrichtung (10) nach Anspruch 1, wobei der Körperadapter (14) eine innere Umfangswand (28) zum vereinenden Eingriff mit einem Auslasskegel (30) des Spritzenkörpers (26) umfasst.

7. Selbstsperrvorrichtung (10) nach einem beliebigen der vorangehenden Ansprüche in Kombination mit einer Spritze (11).

## Revendications

1. Dispositif autobloquant (10) destiné à être utilisé avec une seringue hypodermique stérile à usage unique (11) composé de :
(a) un adaptateur tubulaire (14) configuré pour être fixé à une extrémité de sortie (32) d'un tube (26) de la seringue, la dite fixation étant externe au tube,
(b) un raccord d'aiguille (16) fixé à l'adaptateur tubulaire (14) et ayant une aiguille hypodermique (36) fixée sur elle,
(c) l'adaptateur tubulaire (14) et le raccord d'aiguille (16) formant un assemblage (12) ayant un axe longitudinal, l'assemblage ayant une cavité (38) avec un passage pour une circulation de liquide à partir d'une entrée (34) de l'assemblage vers l'aiguille (36), la cavité (38) ayant une section (66) qui est à l'intérieur du raccord d'aiguille (16) et à l'extérieur de l'adaptateur de tube (14), l'adaptateur tubulaire ayant une partie cylindrique de blocage de l'anneau (47) au niveau d'une extrémité de celui-ci qui s'engage sur le raccord d'aiguille (16),
(d) un anneau d'étanchéité élastiquement déformable (40) dans la cavité (38), l'anneau d'étanchéité (40) ayant une partie de base annulaire (44) et une partie d'étanchéité s'étendant vers l'intérieur (48) ayant une ouverture (50) à l'intérieur, la partie de base annulaire (44) étant ajustée dans la partie de blocage de l'anneau (47) de l'adaptateur tubulaire (14),
(e) un élément d'étanchéité (42) dans la cavité (38) ayant une tête (54) et un axe (52), l'élément d'étanchéité (42) étant mobile dans la cavité (38) dans une direction longitudinale entre une première position dans laquelle la tête (54) de l'élément d'étanchéité (42) est d'un côté de la partie d'étanchéité (48) de l'anneau faisant face à l'entrée (34) de l'assemblage (12) et une deuxième position dans laquelle la tête (54) de l'élément d'étanchéité (42) est d'un côté de la partie d'étanchéité (48) de l'anneau faisant face à l'aiguille (36) et à l'intérieur de la section (66) de la cavité (38) qui est à l'intérieur du raccord d'aiguille (36), et
(f) la partie d'étanchéité (48) de l'anneau (40) étant configurée pour arrêter le mouvement de l'élément d'étanchéité (42) entre la deuxième position et la première position et pour former un joint avec la tête (54), quand l'élément d'étanchéité (42) est dans la deuxième position, contre une circulation de liquide dans la direction de l'aiguille vers l'entrée (34) de l'assemblage.

2. Dispositif autobloquant (10) selon la revendication 1, **caractérisé en ce que** l'adaptateur tubulaire (14) est un adaptateur tubulaire à vis Luer.

3. Dispositif autobloquant (10) selon la revendication 1, **caractérisé en ce que** l'adaptateur tubulaire (14) est un adaptateur tubulaire à glissement Luer.

4. Dispositif autobloquant (10) selon l'une quelconque des revendications précédentes, comprenant en outre des nervures (18) et rainures (20) d'interconnexion pour fixer l'adaptateur de corps (14) et le raccord d'aiguille (16) l'un à l'autre.

5. Dispositif autobloquant (10) selon la revendication 1, **caractérisé en ce que** l'adaptateur tubulaire (14) inclut une paroi circonférentielle externe (22) pour verrouiller l'engagement avec un cône Luer (24) du corps de la seringue (26).

6. Dispositif autobloquant (10) selon la revendication 1, **caractérisé en ce que** l'adaptateur tubulaire (14) inclut une paroi circonférentielle interne (28) pour une prise appariée avec un cône de sortie (30) du corps de la seringue (26).

7. Dispositif autobloquant (10) selon l'une quelconque des revendications précédentes, en combinaison avec une seringue (11).
